# EUROPEAN PATENT SPECIFICATION

(11) **EP 0 660 688 B1**
(45) Date of publication and mention of the grant of the patent: **05.12.2001**
(21) Application number: 93919773.7
(22) Date of filing: 25.08.1993
(51) Int. Cl.: A61B 17/12

(54) **DEVICE FOR STERILE PACKAGING OF MEDICAL EQUIPMENT**
VORRICHTUNG ZUM STERILEN VERPACKEN VON MEDIZINISCHEN ARTIKELN
DISPOSITIF DE CONDITIONNEMENT STERILE D'UN ARTICLE MEDICAL

(30) Priority: 28.08.1992 SE 9202484
(43) Date of publication of application: 05.07.1995
(73) Proprietor: RADI MEDICAL SYSTEMS AB, 754 50 Uppsala (SE)
(72) Inventor: AKERFELDT, Dan, S-755 92 Uppsala (SE)
(74) Representative: Lindgren, Anders
(86) International application number: SE9300705
(87) International publication number: WO9405221

(56) References cited:
- EP-A- 0 462 088
- US-A- 4 224 945
- US-A- 4 233 980

## Description

The present invention relates to sterile packaging of medical equipment, and in particular to a device for the simplification of manual handling of parts which shall be sterile when put in contact with an area on the body of a patient, for example, a puncture site or the like.

The invention is especially suitable for use with inflatable cushions which are used for femoral compression by means of a femoral compressor as described in Swedish patent applications no. 9002077-7 and 9003271-5.

The known femoral compressor (according to Swedish patent applications no. 9002077-7 and 9003271-5) is supplied as one unit (arch and air cushion) in a sterile package which is opened just prior to application onto the patient. The disadvantage with this construction is that the manufacturing and transport costs are high, and that the waste, i.e. the entire unit, is bulky. For possible reuse, the entire compressor must be resterilised, in e.g. an autoclave, in which case the device's large volume is a disadvantage.

The greatest disadvantage with the known femoral compressor is that there is a risk that the contact surface of the permanently mounted air cushion may be contaminated between the moment that the sterile compressor is removed from the package and the moment when it is applied, e.g. on the bleeding puncture site.

In addition, the waste (the compressor), because of blood contamination, is considered hazardous waste, with accompanying troublesome handling'of the bulky unit.

In general, sterile, single-use items which are used in health care and especially during surgical procedures, are usually packaged in sealed, gas-permeable pouches (e.g. TYVEC, a du Pont trademark), so that they can be gas sterilized using ethylene oxide gas after the pouches have been sealed. Sterilisation of the sealed item can also be done by means of radiation treatment (electron radiation, that is, beta sterilisation or gamma radiation). A disadvantage with this kind of sterile packaging is that when the part is to be used, it must be removed from the pouch and handled manually, which clearly implies a risk of contamination of the part on the way to the patient, even if the distance is not very great.

In EP-0 462 088 (Radi Medical Systems) there is disclosed a femoral compression device comprising an pressurizing means for bearing against a puncture site in the femoral artery. The pressurising means is integrated in a belt or in a beam comprising two extensions. The entire assembly is provided as a sterile unit, and either discarded in its entirety or reused after sterilisation in an autoclave.

Therefore, there is a need to be able to handle a sterile part in a safe way, so that the risk for contamination is reduced as much as possible. This can be done by using a package which is constructed in such a way that the seal need not be broken until just before the part is placed on the patient. In this way, the part, e.g. an air cushion for a femoral compressor, can be handled manually and positioned on the patient with no particular care being necessary to avoid contamination.

This object is achieved with a device as defined claim 1.

The preferred embodiments are defined in the dependent claims.

Below a detailed description of the preferred embodiments of th invention will be given, applied to a femoral compressor according to Swedish patent applications 9002077-7 and 9003271-5, and with reference to the attached drawings in which:
Figure 1 shows a cross-section of a patient with a femoral compressor of the above-mentioned type attached over a puncture site;
Figure 2 shows a compression arch belonging to the femoral compressor in Figure 1;
Figure 3 shows an embodiment of a sterile lid according to the invention;
Figure 4 shows an air cushion belonging to the femoral compressor in Fig. 1, with a sterile lid according to the invention;
Figure 5 shows in cross-section, and from below, a part of the compression arch in Fig. 2 adapted to rest against the base plate of a replaceable air cushion;
Figure 6 shows the base plate in cross-section and from below; and
Figure 7 shows the air cushion unit in an inflated state.

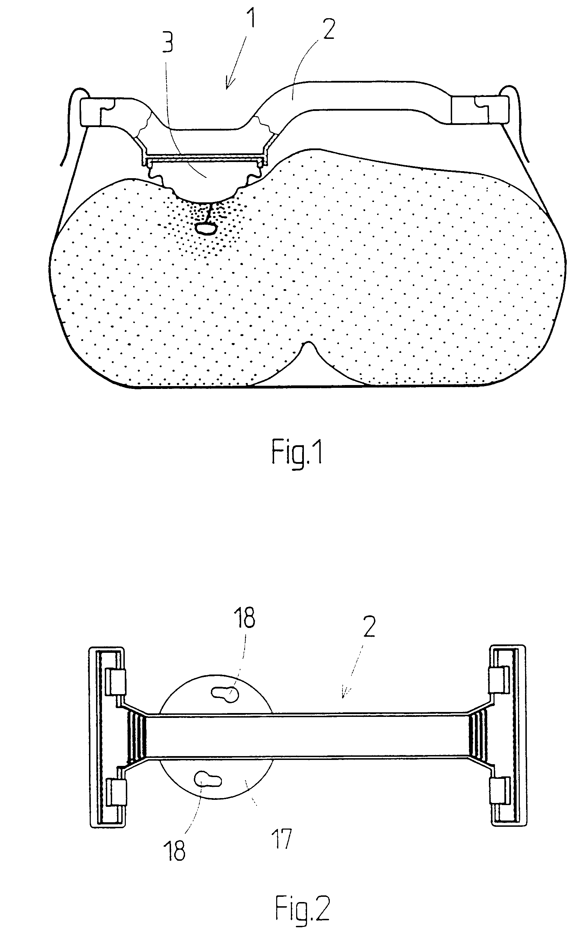

The femoral compressor 1 shown in Figs. 1 and 2 consists of an arch 2 to which an inflatable air cushion 3 is permanently attached. This arch 3 is applied to the puncture site in the femoral artery in order to stop bleeding and is inflated to a suitable pressure using, for example, a manual inflator. Therefore, there is a risk of infection if the air cushion should be contaminated with infectious material.

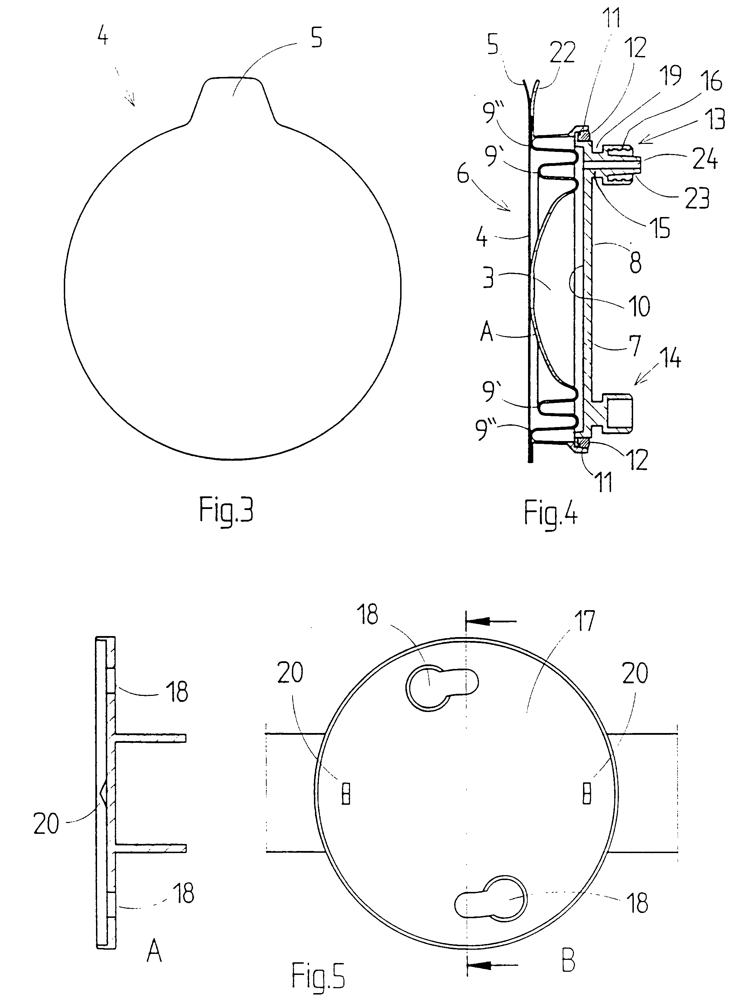

Figure 3 shows a sterile lid 4 according to the invention, meant to be used for sealing the air cushion 3 which is part of the air cushion unit 6 (described below). The lid provides a protective element 4, and includes a primary base layer of spray fiber material (spun bonded), or of a gas permeable, or non-gas permeable polymer material, and a second layer of e.g. melt-glue or of a plastic material which is meltable, so that element 4 is fusible with the above-mentioned periphery.

A suitable, commercially available material for the lid is TYVEC (registered trademark of du Pont).

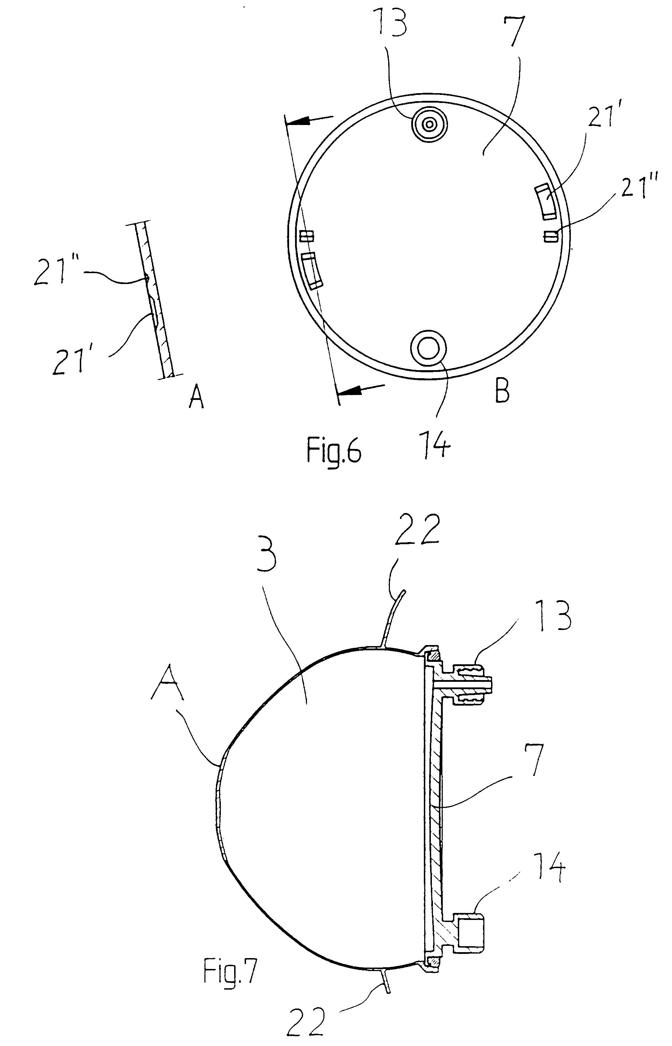

Below the replaceable air cushion unit will be described in detail, with reference to Fig. 4.

The replaceable unit of single use type, is generally shown with the reference number 6, and includes a base plate 7, the upper side 8 of which is attached to the femoral compressor's 1 arch 2. This attachment is achieved by means of a "snap attachment" which will be described in detail below.

On the base plate there is an inflatable air cushion 3 which is mounted by gluing or fusing along the base plate's 7 periphery. The material of which the cushion is made is, seen from a cross sectional view, folded 9', 9" so that the air cushion when not inflated, that is, as it is packaged, takes up as little volume as possible.

Along the periphery of the base plate is a groove 11, wherein melt-glue 12 is placed and after heat and pressure treatment in manner well known to the skilled man, this makes for an airtight sealing of the air cushion 3 against the base plate 7. Two attachment elements 13, 14, stick out from the upper side 8 of the plate 7, said attachment elements 13, 14 being designed so that their end 15 which connects to the base plate 7 is smaller in diameter than their end 16 which is not connected to the baseplate 7. A web or waist 19 is therefore formed between the baseplate and the thicker end piece 16.

The thicker ends 16 of these attachment elements 13 and 14 are designed to fit precisely into the notches 18 of connecting part 17 shown in Fig. 5. These notches have a narrower part 18' which stretches mainly along a line concentrical to the periphery, and has a width equivalent in diameter to the narrower parts 15 of the connecting elements 13, 14. Furthermore, the connecting parts' 17 thickness corresponds to the length of the waist 19. Therefore, if the replaceable air cushion unit's connecting element is fit into the arch's notches 18 and thereafter turned counter-clockwise, then base plate 7 will fit tightly against the connecting part 17.

Furthermore, connecting part 17 is equipped with a protruding element 20, which "snaps" into the corresponding notches 21" in base plate 7 when cushion part 6 is twisted into place. Thus unit 6 is locked to arch 2 and is prevented from loosening from it. In the shown embodiment, the protruding elements 20 are triangular. On the base plate, there are also notches 21' into which the element 20 slides when the air cushion unit 6 is initially placed against the connecting part 17 and the attachment elements 13 and 14 are fit into the larger (wider) part of the notches 18. When the unit 6 is then twisted into place, the element 20 is moved over the part between the notches 21' and 21", whereby the baseplate 7 gives somewhat. Thereafter, the element 20 falls into place in the notches 21", and thereby a locked position is reached and the desired "snap attachement" is achieved.

Alternatively, the protruding element 20 can be attached to the base plate 7, and the notches 21' and 21" can be attached to the connecting part 17.

One of the connecting elements 13 is designed so that it forms an attachment of LUER-LOCK™ type 23. There is a hole 24 in connecting element 13 through which air can be pumped in order to inflate air cushion 3 after the femoral compressor 1 has been placed on a patient.

In order to ensure that the risk of contaminating the contact surface A of air cushion 3 is reduced as much as possible, it is suggested according to the present invention, that a lid (see Fig. 4), preferably made of a "spun bonded" material (spray fiber material), (e.g. TYVEC^{R} from du Pont) equipped with a melt-glue layer, or a layer of other material which can be melted so that a seal is formed, is attached to the air cushion 3. The lid 4 is fused or glued along the periphery of the uninflated cushion 3 (see Fig. 3). The fusing is done by using heat and pressure, typically approximately 130°C and 400 kPa. The fusing time is typically approximately 2 s. The desired peel force should be 0.17 - 0.47 N/mm (4.5 - 12 N/inches), preferably 0.31 N/mm (8 N/inch), which is a suitable balance between good strength and unintentional opening and ease of opening the lid by hand. The lid is equipped with a tab 5 which makes it easy to remove the lid from the edge. The stripping force is typically 0.31 N/mm (8 N/inch).

After the lid 4 has been attached, the air cushion unit 6 is sterilised by e.g. radiation with high energy radiation or by gas sterilisation.

When using a femoral compressor with replaceable air cushion unit 6 of the type described, the air cushion unit 6 is first mounted onto the arch 2. Thanks to the lid 4 which protects the contact surface A on air cushion 3, the nurse or physician who applies the compressor to the patient does not need to be as careful, with regard to the manual handling of the entire instrument, as with the known device of Swedish patent applications no. 9002077-7 and 9003271-5. The unit 6 is mounted as described by putting the attachment elements 13, 14 into the notches 18, whereupon the unit is turned clockwise until it "snaps" into place thanks to the protruding elements' 20 working together with the notches 21 in connecting part 17. The femoral compressor 1 can now be taken to the patient, and immediately before application, the lid 4 is easilily torn off by gripping the tab 5 and pulling diametrically over the air cushion. The stripping force is slight enough that this shall not cause any difficulties.

After completed compression, that is when bleeding no longer occurs, the compressor is removed and the single-use air cushion unit 6 is disassembled by turning it counter-clockwise and pulling it out from the notches 18, whereupon it is discarded as waste. To avoid touching the blood-contaminated air cushion, the unit 6 can be loosened from the arch by turning the air cushion unit from behind using the protruding attachment elements 13 and 14.

## Claims

1. An air cushion unit (6) of single use type suitable for use together with a femoral compressor (1), said air cushion unit comprising
a base plate (7) and an inflatable air cushion (3) attached to the base plate (7), said air cushion (3) being folded (9', 9") when not inflated;
an airtight sealing of the air cushion (3) against the base plate (7);
**characterised by**
attachment means (13, 14) provided on the base plate (7) and protruding therefrom, the end (15) of said protruding attachment means (13, 14) which connects to the base plate (7) having a smaller diameter than the end (16) thereof which is not connected to the base plate (7), so as to form a waist (19) between the base plate (7) and the thicker end piece (16), and
means for sterile protection of the unit.

2. The air cushion unit as claimed in claim 1, comprising a shielding element (4) of a flexible material, sealed along the periphery of said air cushion (3).

3. The air cushion unit as claimed in claim 2, wherein the shielding element (4) is equipped with a tab (5), which can be gripped with the fingers and used to pull off the sealing element (4).

4. The air cushion unit as claimed in claims 2 or 3, comprising a flange (22) running along the edge of the cushion to which the shielding element (4) is fused or glued so that it seals the air cushion (3).

5. The air cushion unit as claimed in any of claims 2-4, wherein the shielding element (4) comprises a first and a second layer, and wherein the second layer is made of melt-glue or a plastic material which is meltable, and wherein the element (4) is melt-fused onto the above-mentioned periphery, to form a seam.

6. The air cushion unit as claimed in claim 5, wherein the first layer is made of spun bonded material, or of gas permeable or gas permeable polymer material.

7. The air cushion unit as claimed in any of claims 5 or 6, wherein the stripping force in said seam is 0.17 - 0.47 N/mm (4.5 - 12 N/inch), preferably 0.31 N/mm (8 N/inch).

8. The air cushion unit as claimed in any preceding claim, which is sterilized after the shielding element (4) has been attached.

## Patentansprüche

1. Einwegluftkisseneinheit (6), geeignet zur Verwendung zusammen mit einer Femoralkompresse (1), wobei die Luftkisseneinheit umfasst:
eine Basisplatte (7) und ein aufblasbares Luftkissen (3), das an der Basisplatte (7) angebracht ist, wobei das Luftkissen (3) gefaltet (9', 9") ist, wenn es nicht aufgeblasen ist;
eine luftdichte Abdichtung des Luftkissens (3) gegen die Basisplatte (7);
**gekennzeichnet durch**
Anbringmittel (13, 14), die auf der Basisplatte (7) vorgesehen sind und davon vorstehen, wobei das Ende (15) der vorspringenden Anbringmittel (13, 14), das mit der Basisplatte (7) verbunden ist, einen kleineren Durchmesser hat als das Ende (16) davon, das nicht mit der Basisplatte (7) verbunden ist, so dass eine Verengung (19) zwischen der Basisplatte (7) und dem dickeren Endstück (16) geformt wird, und
Mittel zum sterilen Schutz der Einheit.

2. Luftkisseneinheit nach Anspruch 1, umfassend ein Abschirmelement (4) aus flexiblem Material, das entlang des Randes des Luftkissens (3) verschlossen ist.

3. Luftkisseneinheit nach Anspruch 2, wobei das Abschirmelement (4) mit einem Fortsatz (5) versehen ist, der mit dem Finger ergriffen werden kann und zum Abziehen des Dichtelements verwendet werden kann.

4. Luftkisseneinheit nach Anspruch 2 oder 3, umfassend einen Flansch (22), der entlang des Rands des Kissens läuft, an dem das Abschirmelement (4) verschweißt oder geklebt ist, so dass es das Luftkissen (3) verschließt.

5. Luftkisseneinheit nach einem der Ansprüche 2 bis 4, wobei das Abschirmelement (4) eine erste und eine zweite Schicht umfasst, und wobei die zweite Schicht aus einem Schmelzkleber oder einem Kunststoffmaterial ist, das schmelzbar ist, und wobei das Element (4) auf den oben erwähnten Rand durch Schmelzverbindung angebracht ist, so dass eine Naht geformt wird.

6. Luftkisseneinheit nach Anspruch 5, wobei die erste Schicht aus einem Spunbondingmaterial gefertigt ist oder aus gasdurchlässigem oder gasdurchlässigem Polymermaterial.

7. Luftkisseneinheit nach einem der Ansprüche 5 oder 6, wobei die Abziehkraft in der Naht 0,17 bis 0,47 N/mm (4,5 - 12 N/inch) ist, vorzugsweise 0,31 N/mm (8 N/inch).

8. Luftkisseneinheit nach einem der vorhergehenden Ansprüche, die sterilisiert ist, nachdem das Abschirmelement (4) angebracht worden ist.

## Revendications

1. Unité (6) à coussin d'air du type à usage unique convenant à une utilisation avec un compresseur fémoral (1), ladite unité à coussin d'air comportant
une plaque de base (7) et un coussin d'air gonflable (3) attaché à la plaque de base (7), ledit coussin d'air (3) étant plié (9', 9") lorsqu'il n'est pas gonflé ;
un scellement étanche à l'air du coussin d'air (3) contre la plaque de base (7) ;
**caractérisée par**
des moyens d'attache (13, 14) prévus sur la plaque de base (7) et qui en font saillie, l'extrémité (15) desdits moyens d'attache (13, 14) en saillie qui est reliée à la plaque de base (7) ayant un diamètre inférieur à celui de leur extrémité (16) qui n'est pas reliée à la plaque de base (7), afin de former un rétrécissement (19) entre la plaque de base (7) et la pièce d'extrémité plus épaisse (16), et
des moyens pour la protection stérile de l'unité.

2. Unité à coussin d'air selon la revendication 1, comportant un élément (4) de protection en matière flexible, scellé le long de la périphérie dudit coussin d'air (3).

3. Unité à coussin d'air selon la revendication 2, dans laquelle l'élément de protection (4) est équipé d'une languette (5), laquelle peut être prise avec les doigts et utilisée pour enlever le tirant l'élément de scellement (4).

4. Unité à coussin d'air selon les revendications 2 ou 3, comportant un rebord (22) s'étendant le long du bord du coussin auquel l'élément de protection (4) est soudé ou collé afin de sceller de façon étanche le coussin d'air (3).

5. Unité à coussin d'air selon l'une quelconque des revendications 2 à 4, dans laquelle l'élément de protection (4) comporte des première et seconde couches, et dans laquelle la seconde couche est formée d'une colle fusible ou d'une matière plastique qui peut fondre, et dans laquelle l'élément (4) est soudé par fusion sur la périphérie mentionnée précédemment, de façon à former un joint.

6. Unité à coussin d'air selon la revendication 5, dans laquelle la première couche est formée d'une matière non tissée, ou d'une matière polymère perméable aux gaz.

7. Unité à coussin d'air selon l'une quelconque des revendications 5 ou 6, dans laquelle la force d'arrachement dudit joint est de 0,17 à 0,47 N/mm (4,5 à 12 N/inch), avantageusement de 0,31 N/mm (8 N/inch).

8. Unité à coussin d'air selon l'une quelconque des revendications précédentes, qui est stérilisée après que l'élément de protection (4) a été attaché.
